# EUROPÄISCHE PATENTSCHRIFT

(11) **EP 0 418 633 B1**
(45) Veröffentlichungstag und Bekanntmachung des Hinweises auf die Patenterteilung: **02.03.1994**
(21) Anmeldenummer: 90116927.6
(22) Anmeldetag: 04.09.1990
(51) Int. Cl.: C07C 309/39

(54) **Verfahren zur Herstellung aromatischer (Di)Chlorsulfonsäuren**
Process for the preparation of aromatic (di)chlorosulfonic acids
Procédé pour la préparation d'acides (di) chlorosulfoniques aromatiques

(30) Priorität: 16.09.1989 DE 3930994
(43) Veröffentlichungstag der Anmeldung: 27.03.1991
(73) Patentinhaber: BAYER AG, 51368 Leverkusen (DE)
(72) Erfinder: Blank, Heinz Ulrich, Dr., D-5068 Odenthal (DE); Heinz, Uwe, Dr., D-5000 Köln 80 (DE)

(56) Entgegenhaltungen:
- EP-A- 0 057 889
- DE-B- 1 279 672
- DE-C- 312 959
- DE-C- 491 220
- FR-A- 2 297 836
- US-A- 4 131 619
- BERNHARD PRAGER ET AL.: "BEILSTEINS HANDBUCH DER ORGANISCHEN CHEMIE" 4. AUFLAGE, 11. BAND, 1928, VERLAG JULIUS SPRINGER, BERLIN, S. 56, 57
- Garrick, A.R., Zeitschrift für Chemie, 1869, Seiten 549-551

## Beschreibung

Die vorliegende Erfindung betrifft ein Verfahren zur Herstellung von aromatischen (Di)Chlorsulfonsäuren. Solche einfach oder zweifach chlorierten aromatischen Sulfonsäuren sind vielfältig verwendbare Zwischenprodukte, beispielsweise zur Herstellung von Farbstoffen oder Pflanzenschutzmitteln.

Aromatische Sulfonsäuren können nach bisherigen Verfahren beispielsweise so chloriert werden, daß die Sulfonsäure entweder in einem polaren Lösungsmittel, wie Wasser oder Schwefelsäure, oder in einem inerten Lösungsmittel bei gleichzeitiger Anwesenheit eines Katalysators mit Chlor in Kontakt gebracht werden. Häufig werden die Sulfonierung des zugrundeliegenden Aromaten und die Chlorierung nacheinander im gleichen Reaktionsansatz durchgeführt. Beispiele für die zuerst genannte Ausführungsart sind EP 57 889 oder DE-OS 25 01 899 zu entnehmen. Als Beispiel für die zweite genannte Ausführungsform sei US 4.131.619 genannt. Beide genannten Ausführungsformen entsprechen dem ionischen Mechanismus dieser Chlorierung. Beide Varianten haben den Nachteil, daß große Mengen verunreinigter Lösungsmittel anfallen, die entweder entsorgt oder zur Wiederverwendung aufgearbeitet werden müssen; bei gleichzeitiger Verwendung eines Katalysators ist dieser zusätzlich zu behandeln.

Aus Zeitschrift für Chemie, Vol. 12 (1869), S. 549 bis 551 ist es weiter bekannt, durch gemeinsames Erhitzen von äquimolaren Mengen Brom und Benzolsulfonsäure in zugeschmolzenen Röhren auf 100°C 3-Brom-benzolsulfonsäure zu erhalten. Eine Ausbeute wird nicht angegeben; die Reaktion wird vielmehr als nicht glatt beschrieben, wobei ein Teil der Benzolsulfonsäure unverändert bleibt, während ein anderer Teil sich in freie Schwefelsäure und feste, kristallisierende, aber nicht näher beschriebene Benzolsubstitutionsprodukte zersetzt.

Es bestand daher ein Bedürfnis, ein Verfahren zur Verfügung zu haben, das die Chlorierung aromatischer Sulfonsäuren ohne Verwendung eines Lösungsmittels und eines Katalysators erlaubt.

Es wurde gefunden, daß aromatische Sulfonsäuren ohne Lösungsmittel oder Katalysator chloriert werden können, wenn man die Umsetzung in der Schmelze einer solchen aromatischen Sulfonsäure durchführt.

Die Erfindung betrifft daher ein Verfahren zur Herstellung aromatischer (Di)Chlorsulfonsäuren durch Umsetzung aromatischer Sulfonsäuren mit Chlor, das dadurch gekennzeichnet ist, daß man zur Umsetzung in die Schmelze der aromatischen Sulfonsäuren Chlor in elementarer Form oder in Form einer Verbindung, die unter den Verfahrensbedingungen elementares Chlor abgibt, einleitet.

Zur Umsetzung wird Chlor entweder in elementarer Form oder in der Form einer Verbindung, die unter den erfindungsgemäßen Bedingungen elementares Chlor abgibt, in die Schmelze einer aromatischen Sulfonsäure eingeleitet. Als Verbindung, die unter den erfindungsgemäßen Bedingungen Chlor abgibt, sei beispielsweise Sulfurylchlorid genannt. In bevorzugter Weise wird mit elementarem Chlor gearbeitet. Das elementare Chlor oder die elementares Chlor abgebende Verbindung kann entweder gasförmig oder flüssig in das Reaktionsgemisch eingeleitet werden. Das elementare Chlor wird bevorzugt gasförmig eingeleitet. Zur zügigen Durchführung der Chlorierung durch Erhöhung der Konzentration des Chlors kann es zweckmäßig sein, das Chlor mit erhöhtem Druck einzuleiten. So kann beispielsweise das elementare Chlor sowohl mit atmosphärischem Druck als auch vorteilhafterweise mit einem Druck zwischen 1 und 6 bar eingeleitet werden. Die Chlorierung führt je nach der Menge des Chlorierungsmittels zu einfach oder zweifach chlorierten aromatischen Sulfonsäuren. Es werden 90 bis 130 Mol-% Chlorierungsmittel je einzuführendem Chlorsubstituenten eingesetzt. Eine zweifache Chlorierung kann auch nacheinander in zweistufiger Reaktion erfolgen. In bevorzugter Weise wird eine Einfachchlorierung durchgeführt.

Das erfindungsgemäße Verfahren wird in der Schmelze der zugrundeliegenden aromatischen Sulfonsäure durchgeführt. Die Reaktionstemperatur liegt daher oberhalb der Schmelztemperatur dieser aromatischen Sulfonsäure. In vielen Fällen liegt die Reaktionstemperatur im Bereich von 40-200°C, bevorzugt im Bereich von 50-120°C. Im höheren Temperaturbereich wird bevorzugt nur im Falle hochschmelzender Sulfonsäuren gearbeitet.

Erfindungsgemäß können aromatische Sulfonsäuren der Formel 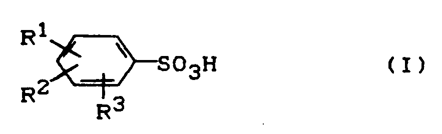
umgesetzt werden, in der
- R¹: Wasserstoff, geradkettiges oder verzweigtes C₁-C₂₀-Alkyl, C₃-C₈-Cycloalkyl, Benzyl, substituiertes Benzyl, Phenyl, substituiertes Phenyl, geradkettiges oder verzweigtes C₁-C₂₀-Alkoxy, Benzyloxy, substituiertes Benzyloxy, Phenoxy, substituiertes Phenoxy, Nitro oder Cyano bedeutet,
- R²: für Wasserstoff, Methyl oder Ethyl steht und
- R³: Wasserstoff, Methyl, Ethyl, Fluor, Chlor oder Brom bedeutet,
wobei weiterhin R¹ und R² gemeinsam einen anellierten Benzolring oder substituierten Benzolring, einen anellierten aromatischen oder nicht aromatischen 5- oder 6-gliedrigen Heterocyclus oder Trimethylen oder Tetramethylen bedeuten können.

Bevorzugt können Sulfonsäuren der Formel 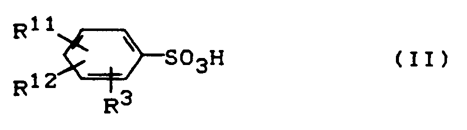
umgesetzt werden, in der
- R¹¹: Wasserstoff, geradkettiges oder verzweigtes C₁-C₂₀-Alkyl, geradkettiges oder verzweigtes C₁-C₂₀-Alkoxy, Cyclopropyl, Cyclopentyl, Cyclohexyl, Benzyl, substituiertes Benzyl, Phenyl oder substituiertes Phenyl bedeutet,
- R¹²: für Wasserstoff, Methyl oder Ethyl steht und
- R³: die angegebene Bedeutung hat,
wobei weiterhin R¹¹ und R¹² gemeinsam einen anellierten Benzolring oder substituierten Benzolring, Trimethylen oder Tetramethylen bedeuten können.

Besonders bevorzugt können Sulfonsäuren der Formel 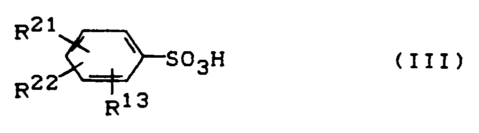
umgesetzt werden, in der
- R²¹: Wasserstoff oder geradkettiges oder verzweigtes C₁-C₄-Alkyl bedeutet und
- R²² und R¹³: unabhängig voneinander für Wasserstoff, Methyl oder Ethyl stehen.

Die aromatische Sulfonsäure kann hierbei als solche, in vielen Fällen auch in der Form der Sulfierschmelze eingesetzt werden, wie sie bei der Sulfierung der zugrundeliegenden aromatischen Verbindung mit SO₃ erhalten wird. Schwefelsäure kann hierbei in einer Menge von 0,1-25 Gew.-%, bezogen auf die Summe von Sulfonsäure und Schwefelsäure, zugegen sein.

Weiterhin kann es in vielen Fällen sinnvoll sein, in Gegenwart von bis zu 10 Gew.-% Wasser, bezogen auf die umzusetzende Sulfonsäure, zu arbeiten. Dies gilt beispielsweise für den Einsatz der Sulfonsäuren in Form ihrer Hydrate. Weder die Schwefelsäure noch das Wasser sind für die Durchführbarkeit des erfindungsgemäßen Verfahrens erforderlich.

In weiterhin bevorzugter Weise wird als aromatische Sulfonsäure 4-Toluolsulfonsäure oder eine hauptsächlich 4-Toluolsulfonsäure enthaltende Sulfierschmelze eingesetzt.

Geradkettiges oder verzweigtes C₁-C₂₀-Alkyl ist beispielsweise Methyl, Ethyl, Propyl, Isopropyl, Butyl, Isobutyl, tert.-Butyl, eines der isomeren Hexyle, Octyle, Decyle, Dodecyle, Hexadecyle oder Eicosyle. Geradkettiges oder verzweigtes C₁-C₂₀-Alkoxy ergibt sich aus dem genannten C₁-C₂₀-Alkyl durch Aufnahme eines O-Atoms. Bevorzugt ist C₁-C₁₂-Alkyl bzw. C₁-C₁₂-Alkoxy, besonders bevorzugt ist C₁-C₈-Alkyl bzw. C₁-C₈-Alkoxy, ganz besonders bevorzugt ist C₁-C₄-Alkyl bzw. C₁-C₄-Alkoxy.

Substituiertes Benzyl, Phenyl, Benzyloxy oder Phenoxy oder ein substituierter anellierter Benzolring trägt eine Sulfonsäuregruppe oder R³.

Für den Fall, daß R¹ und R² gemeinsam einen anellierten 5- oder 6-gliedrigen Heterocyclus bilden, gelangt man, gemeinsam mit dem die Reste R¹ und R² tragenden Benzolkern, in die Reihe der benzokondensierten aromatischen oder durch (Teil)Hydrierung entstehenden nichtaromatischen Heterocyclen mit einem oder zwei Heteroatomen aus der Gruppe N, O und S, wie Indol, Indolenin, Cumaron, Thionaphthen, Benzimidazol, Benzoxazol, Benzthiazol, Chinolin, Isochinolin, Chromen, Chroman, Benzoxazin.

Für den Fall, daß R¹ und R² gemeinsam Trimethylen oder Tetramethylen bilden, gelangt man in die Indan- bzw. Tetralinreihe.

Das Chlor tritt bevorzugt in die Position ein, die nach den dem Fachmann bekannten allgemeinen Regeln der elektrophilen aromatischen Substitution begünstigt ist. Bei 4-Sulfotoluol ist dies die 2-Position. Beim Einsatz einer Sulfierschmelze, die beispielsweise durch Umsetzung von Toluol mit SO₃ entstanden ist, enthält diese Sulfierschmelze neben der 4-Toluolsulfonsäure auch wechselnde Anteile von 2- und 3-Toluolsulfonsäure. In einem solchen Fall werden alle drei isomeren Chlortoluolsulfonsäuren gebildet. Sie können durch übliche Reinigungs- bzw. Aufarbeitungsmethoden in reiner Form isoliert werden.

### Beispiel 1

### 2-Chlor-4-toluol-sulfonsäure aus 4-Toluolsulfonsäure

Aus 241,5 g 4-Toluolsulfonsäure-Hydrat wurde mit Benzol das Wasser ausgekreist. Nachdem kein Wasser mehr abgeschieden wurde, wurde das Benzol abdestilliert. Letzte Reste Benzol wurden im Stickstoffstrom ausgetrieben. Man erhielt 189,9 g (1,10 Mol) 4-Toluolsulfonsäure. Sodann wurde bei 60°C so lange Chlor eingeleitet, bis die Gewichtszunahme des Ansatzes der theoretisch erwarteten (39 g) entsprach. Nach Austreiben von restlichem Chlor und Chlorwasserstoff im Stickstoffstrom betrug die tatsächliche Gewichtszunahme des Ansatzes 37,4 g (1,06 Mol Chlor). Die Hochleistungs-Flüssigkeitschromatographie-Analyse (HPLC = High Performance Liquid Chromatography) ergab einen Gehalt von 88,3 % (88,3 % der theoretischen Ausbeute) 2-Chlor-4-toluolsulfonsäure neben 1,72 % (1,72 % der theoretischen Ausbeute) 3-Chlor-4-toluolsulfonsäure und 1,3 % (1,56 % der eingesetzten Sulfonsäure) 4-Toluolsulfonsäure.

### Beispiel 2

### Chlorieren einer Sulfierschmelze

In 256,7 g einer Sulfierschmelze, wie sie bei der Sulfierung von Toluol mit Schwefeltrioxid erhalten worden war (enthielt 72,9 % 4-Toluolsulfonsäure und 12,8 % 2-Toluolsulfonsäure), wurde bei 65°C so lange Chlor eingeleitet, bis die theoretisch erwartete Gewichtszunahme des Ansatzes erreicht war. Anschließend wurden restliches Chlor und Chlorwasserstoff mit Stickstoff ausgetrieben. Der Ansatz wog nun 306,5 g. HPLC-Analyse ergab einen Gehalt von 62,1 % 2-Chlor-4-toluolsulfonsäure, das sind 85 % der theoretischen Ausbeute. Daneben wurden 8,1 % 4-Chlor-2-toluolsulfonsäure und 3,6 % (5 % der eingesetzten Sulfonsäure) 4-Toluolsulfonsäure nachgewiesen.

## Patentansprüche

1. Verfahren zur Herstellung aromatischer (Di)Chlorsulfonsäuren durch Umsetzung aromatischer Sulfonsäuren mit Chlor, dadurch gekennzeichnet, daß man zur Umsetzung in die Schmelze der aromatischen Sulfonsäuren Chlor in elementarer Form oder in Form einer Verbindung, die unter den Verfahrensbedingungen elementares Chlor abgibt, einleitet.

2. Verfahren nach Anspruch 1, dadurch gekennzeichnet, daß elementares Chlor, eingeleitet wird.

3. Verfahren nach Anspruch 2, dadurch gekennzeichnet, daß das elementare Chlor mit einem Druck zwischen 1 und 6 bar eingeleitet wird.

4. Verfahren nach Anspruch 1, dadurch gekennzeichnet, daß 90-130 Mol-% Chlorierungsmittel je einzuführendem Chlorsubstituenten eingesetzt werden.

5. Verfahren nach Anspruch 1, dadurch gekennzeichnet, daß die Umsetzung bei 40-200°C, bevorzugt bei 50-120°C, durchgeführt wird.

6. Verfahren nach Anspruch 1, dadurch gekennzeichnet, daß als aromatische Sulfonsäuren solche der Formel 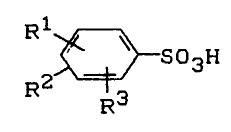 umgesetzt werden, in der
R¹ Wasserstoff, geradkettiges oder verzweigtes C₁-C₂₀-Alkyl, C₃-C₈-Cycloalkyl, Benzyl, substituiertes Benzyl, Phenyl, substituiertes Phenyl, geradkettiges oder verzweigtes C₁-C₂₀-Alkoxy, Benzyloxy, substituiertes Benzyloxy, Phenoxy, substituiertes Phenoxy, Nitro oder Cyano bedeutet,
R² für Wasserstoff, Methyl oder Ethyl steht und
R³ Wasserstoff, Methyl, Ethyl, Fluor, Chlor oder Brom bedeutet,
wobei weiterhin R¹ und R² gemeinsam einen anellierten Benzolring oder substituierten Benzolring, einen anellierten aromatischen oder nicht aromatischen 5- oder 6-gliedrigen Heterocyclus oder Trimethylen oder Tetramethylen bedeuten können.

7. Verfahren nach Anspruch 6, dadurch gekennzeichnet, daß als aromatische Sulfonsäuren solche der Formel 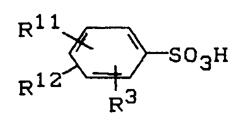 umgesetzt werden, in der
R¹¹ Wasserstoff, geradkettiges oder verzweigtes C₁-C₂₀-Alkyl, geradkettiges oder verzweigtes C₁-C₂₀-Alkoxy, Cyclopropyl, Cyclopentyl, Cyclohexyl, Benzyl, substituiertes Benzyl, Phenyl oder substituiertes Phenyl bedeutet,
R¹² für Wasserstoff, Methyl oder Ethyl steht und
R³ die in Anspruch 6 angegebene Bedeutung hat, wobei weiterhin R¹¹ und R¹² gemeinsam einen anellierten Benzolring oder substituierten Benzolring, Trimethylen oder Tetramethylen bedeuten können.

8. Verfahren nach Anspruch 7, dadurch gekennzeichnet, daß als aromatische Sulfonsäuren solche der Formel 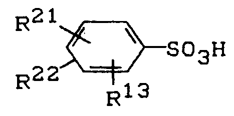 umgesetzt werden, in der
R²¹ Wasserstoff, geradkettiges oder verzweigtes C₁-C₄-Alkyl bedeutet und
R²² und R¹³ unabhängig voneinander für Wasserstoff, Methyl oder Ethyl stehen.

9. Verfahren nach Anspruch 6, dadurch gekennzeichnet, daß die aromatischen Sulfonsäuren als Sulfierschmelze eingesetzt werden, wie sie bei der Sulfierung der zugrundeliegenden aromatischen Verbindungen mit SO₃ erhalten wird.

10. Verfahren nach Anspruch 7, dadurch gekennzeichnet, daß als aromatische Sulfonsäure 4-Toluolsulfonsäure oder eine hauptsächlich 4-Toluolsulfonsäure enthaltende Sulfierschmelze eingesetzt wird.

## Claims

1. Process for the preparation of aromatic (di)chlorosulphonic acids by reacting aromatic sulphonic acids with chlorine, characterized in that, for the reaction, chlorine is passed into the melt of the aromatic sulphuric acids in elemental form or in the form of a compound which releases elemental chlorine under the process conditions.

2. Process according to Claim 1, characterized in that elemental chlorine is passed in.

3. Process according to Claim 2, characterized in that the elemental chlorine is passed in at a pressure of between 1 and 6 bar.

4. Process according to Claim 1, characterized in that 90-130 mol % of chlorinating agent are employed per chlorine substituent to be introduced.

5. Process according to Claim 1, characterized in that the reaction is carried out at 40-200°C, preferably at 50-120°C.

6. Process according to Claim 1, characterized in that the aromatic sulphonic acids reacted are those of the formula 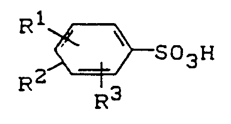 in which
R¹ denotes hydrogen, straight-chain or branched C₁-C₂₀-alkyl, C₃-C₈-cycloalkyl, benzyl, substituted benzyl, phenyl, substituted phenyl, straight-chain or branched C₁-C₂₀-alkoxy, benzyloxy, substituted benzyloxy, phenoxy, substituted phenoxy, nitro or cyano,
R² represents hydrogen, methyl or ethyl and
R³ represents hydrogen, methyl, ethyl, fluorine, chlorine or bromine,
where furthermore R¹ and R² together can denote a fused benzene ring or substituted benzene ring, a fused aromatic or non-aromatic 5- or 6-membered heterocycle, or trimethylene or tetramethylene.

7. Process according to Claim 6, characterized in that the aromatic sulphonic acids reacted are those of the formula 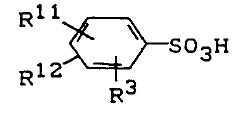 in which
R¹¹ denotes hydrogen, straight-chain or branched C₁-C₂₀-alkyl, straight-chain or branched C₁-C₂₀-alkoxy, cyclopropyl, cyclopentyl, cyclohexyl, benzyl, substituted benzyl, phenyl or substituted phenyl,
R¹² represents hydrogen, methyl or ethyl and R³ has the meaning given in Claim 6,
where furthermore R¹¹ and R¹² together can denote a fused benzene ring or substituted benzene ring, or trimethylene or tetramethylene.

8. Process according to Claim 7, characterized in that the aromatic sulphonic acids reacted are those of the formula 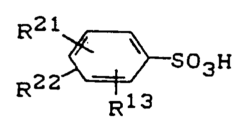 in which
R²¹ denotes hydrogen, straight-chain or branched C₁-C₄-alkyl and
R²² and R¹³ independently of one another represent hydrogen, methyl or ethyl.

9. Process according to Claim 6, characterized in that the aromatic sulphonic acids are employed as sulphonation melt as is obtained in the sulphonation of the basic aromatic compounds with SO₃.

10. Process according to Claim 7, characterized in that the aromatic sulphonic acid employed is 4-toluenesulphonic acid or a sulphonation melt which mainly contains 4-toluenesulphonic acid.

## Revendications

1. Procédé de préparation d'acides (di)chlorosulfoniques aromatiques par réaction d'acides sulfoniques aromatiques avec le chlore, caractérisé en ce que, pour la réaction, on injecte dans la masse fondue de l'acide sulfonique aromatique du chlore à l'état élémentaire ou à l'état de composé libérant du chlore élémentaire dans les conditions observées.

2. Procédé selon la revendication 1, caractérisé en ce que l'on injecte du chlore élémentaire.

3. Procédé selon la revendication 2, caractérisé en ce que l'on injecte le chlore élémentaire sous une pression de 1 à 6 bar.

4. Procédé selon la revendication 1, caractérisé en ce que l'on met en oeuvre de 90 à 130 mol % de l'agent chlorant par substituant chloro à introduire.

5. Procédé selon la revendication 1, caractérisé en ce que la réaction est effectuée à des températures de 40 à 200°C, de préférence de 50 à 120°C.

6. Procédé selon la revendication 1, caractérisé en ce que l'on convertit des acides sulfoniques aromatiques de formule 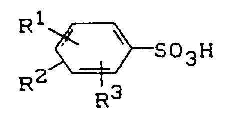 dans laquelle
R¹ représente l'hydrogène, un groupe alkyle à chaîne droite ou ramifiée en C₁-C₂₀, un groupe cycloalkyle en C₃-C₈, benzyle éventuellement substitué, phényle éventuellement substitué, alcoxy à chaîne droite ou ramifiée en C₁-C₂₀, benzyloxy éventuellement substitué, phénoxy éventuellement substitué, nitro ou cyano,
R² représente l'hydrogène, un groupe méthyle ou éthyle, et
R³ représente l'hydrogène, un groupe méthyle, éthyle, le fluor, le chlore ou le brome,
R¹ et R² pouvant également former ensemble un cycle benzénique condensé ou un cycle benzénique substitué condensé, un hétérocycle aromatique ou non aromatique condensé à 5 ou 6 chaînons ou un groupe triméthylène ou tétraméthylène.

7. Procédé selon la revendication 6, caractérisé en ce que l'on convertit des acides sulfoniques aromatiques de formule 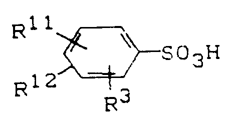 dans laquelle
R¹¹ représente l'hydrogène, un groupe alkyle à chaîne droite ou ramifiée en C₁-C₂₀, alcoxy à chaîne droite ou ramifiée en C₁-C₂₀, cyclopropyle, cyclopentyle, cyclohexyle, benzyle éventuellement substitué, phényle éventuellement substitué,
R¹² représente l'hydrogène, un groupe méthyle ou éthyle, et
R³ a les significations indiquées dans la revendication 6,
R¹¹ et R¹² pouvant également former ensemble un cycle benzénique condensé ou un cycle benzénique substitué condensé, un groupe triméthylène ou tétraméthylène.

8. Procédé selon la revendication 7, caractérisé en ce que l'on convertit des acides sulfoniques aromatiques de formule 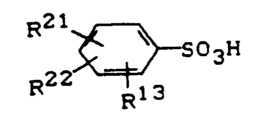 dans laquelle
R²¹ représente l'hydrogène, un groupe alkyle à chaîne droite ou ramifiée en C₁-C₄, et
R²² et R¹³ représentent chacun, indépendamment l'un de l'autre, l'hydrogène, un groupe méthyle ou éthyle.

9. Procédé selon la revendication 6, caractérisé en ce que l'on met en oeuvre les acides sulfoniques aromatiques à l'état de masse de sulfonation telle qu'obtenue à la sulfonation du composé aromatique correspondant par SO₃.

10. Procédé selon la revendication 7, caractérisé en ce que l'on met en oeuvre en tant qu'acide sulfonique aromatique l'acide 4-toluène-sulfonique ou une masse de sulfonation consistant principalement en acide 4-toluène-sulfonique.
